# EUROPEAN PATENT APPLICATION

(11) **EP 4 239 322 A1**
(43) Date of publication of application: **06.09.2023**
(21) Application number: 23154041.0
(22) Date of filing: 30.01.2023
(51) Int. Cl.: G01N 23/04

(54) **X-RAY INSPECTION APPARATUS AND METHOD**

(30) Priority: 01.03.2022 GB 202202836
(71) Applicant: Illinois Tool Works Inc., Glenview IL 60025 (US)
(72) Inventor: ROETON, Adrian, Glenview, 60025 (US); CHENG, Qin, Glenview, 60025 (US); WHYMAN, Stuart, Glenview, 60025 (US)
(74) Representative: HGF

(57) **Abstract**

There is provided an X-ray product inspection apparatus 200 and a method of controlling the X-ray product inspection apparatus. The apparatus 200 comprises an X-ray generator 210 comprising an aperture 212 for outputting an X-ray beam, and a shutter apparatus arranged across the aperture 212. The shutter apparatus comprises a moveable shutter plate 220 having a barrier portion 222 of X-ray attenuating material configured to substantially block the X-ray beam; and an actuator mechanism 230 configured to move the shutter plate 220 between a closed position in which the barrier portion 222 is disposed across the aperture 212 to substantially block the X-ray beam and an open position in which the barrier portion 222 is offset from the aperture 212 to enable at least partial emission of the X-ray beam.

## Description

This invention relates to an X-ray product inspection apparatus, to a method for controlling an X-ray product inspection apparatus and to computer software.

### BACKGROUND

X-ray inspection can be utilised to detect internal features of products which would typically be hidden from view. X-rays are generated, for example by using an X-ray tube located adjacent to the product to be inspected. A detector is disposed at the opposite side of the object and records an image of the X-rays transmitted through the object, either by converting the X-rays into visible light and imaging using an optical camera, or detecting X-rays directly using an X-ray sensor array. As different materials absorb the X-rays differently, the resultant image can be utilised to inspect an internal structure of the product.

X-ray inspection is utilised in various industries for product inspection, such as food production, in which X-ray inspection can be harnessed to detect contaminants within the product such as metal, bone or glass. The X-ray inspection apparatus can be disposed on a production line, to automatically inspect products fed through such as on a conveyor system.

X-rays can be damaging, and thus it can be desired to control the dispersion of the X-rays to minimise scatter outside the X-ray inspection apparatus. Typically, this is ensured using a shield such as a shielding curtain disposed between the X-ray inspection apparatus and the wider production area. Products may then be fed into the X-ray inspection apparatus through the shielding curtain, such as on the conveyor. However, in industries such as food production it can also be desired to avoid contact between the product and any shielding curtain as the product may be uncovered or unwrapped, thus posing a hygiene concern. It can thus be desirable to reduce X-ray scatter in a manner which can be used in an inspection apparatus without a shielding curtain.

It is an aim of the present invention to mitigate one or more of the problems associated with the prior art.

### BRIEF SUMMARY OF THE DISCLOSURE

In accordance with the present inventions there is provided an X-ray product inspection apparatus, a method of controlling an X-ray product inspection apparatus and computer software.

According to a first aspect there is provided an X-ray product inspection apparatus, comprising: an X-ray generator comprising an aperture for outputting an X-ray beam; a shutter apparatus arranged across the aperture. The shutter apparatus comprises: a moveable shutter plate having a barrier portion of X-ray attenuating material configured to substantially block the X-ray beam; and an actuator mechanism configured to move the shutter plate between a closed position in which the barrier portion is disposed across the aperture to substantially block the X-ray beam and an open position in which the barrier portion is offset from the aperture to enable at least partial emission of the X-ray beam. Substantially block may mean to block more than 90%, 98%, or 99% of X-rays in the X-ray beam. Substantially block may alternatively mean that the barrier portion completely blocks X-ray beam.

The X-ray product inspection apparatus may further comprise a controller configured to: determine a presence or absence of a product to be inspected in the X-ray inspection apparatus; and output a control signal to the actuator mechanism to move the shutter plate between the closed position and the open position in dependence on the determination. Optionally, the X-ray product inspection apparatus comprises at least one sensor configured to detect the product in the X-ray inspection apparatus and provide a product signal indicative of the detection to the controller, wherein the controller is configured to determine the presence or absence of the product in dependence on the product signal. Optionally, the controller is configured to: determine the presence of a product in dependence on the detection of the product by the at least one sensor; and determine the absence of a product in dependence on a lack of detection of a product by the at least one sensor for at least a predetermined time. Optionally, the controller is configured to output a control signal to the actuator mechanism to retain the shutter plate in the open position for a predetermined time window in dependence on the determination of the presence of a product. The predetermined time window may be defined as sufficient for the product to pass the X-ray beam following being detected. For example, the predetermined time window may be 5s, 10s or 15s for example. Optionally, the controller is configured to output a control signal to the actuator mechanism to move the shutter plate to the closed position in dependence on the determination of the absence of a product.

The controller may be optionally configured to output a control signal to the X-ray generator to enter a low power state in response to the determination of the absence of a product. The controller may be configured to output a control signal to the X-ray generator to resume a full power state from the low power state in response to the determination of the presence of a product.

Optionally, the movable shutter plate comprises an aperture portion at least partially permeable to the X-ray beam, and in the open position the aperture portion aligns with the aperture to enable at least partial emission of the X-ray beam through the aperture portion. In some examples, the barrier portion may substantially surround the aperture portion. In some examples, the aperture portion may correspond to a shape of the aperture The shutter plate may optionally comprise a filter layer of material across the aperture portion configured to attenuate a portion of the X-ray beam below an energy threshold. For example, the filter layer may be aluminium.

The barrier portion may comprise a layer of brass. In some examples, the barrier portion may further comprise a layer of stainless steel.

Optionally, the shutter apparatus comprises a biasing mechanism configured to bias the shutter plate to the closed position. The biasing mechanism may comprise a spring connected to the shutter plate.

Optionally, the actuator mechanism comprises one or more solenoids which are arranged to move the shutter plate from the closed position to the open position when energised.

Optionally, the shutter apparatus comprises a linear bearing, and wherein the shutter plate comprises an arm portion arranged to move along the linear bearing between the open position and the closed position.

In some examples, the shutter apparatus comprises a proximity sensor arranged to detect whether the shutter plate is in the open position or the closed position.

Optionally, the X-ray generator comprises a collimator for collimating the X-ray beam.

According to another aspect there is provided a computer-implemented method for controlling an X-ray product inspection apparatus comprising an X-ray generator having an aperture for outputting an X-ray beam, the method comprising: determining a presence or absence of a product to be inspected in the X-ray inspection apparatus; and in dependence on the determination, controlling an actuator mechanism to move a shutter plate across the aperture between a closed position in which a barrier portion of the shutter plate is disposed across the aperture to substantially block the X-ray beam and an open position in which the barrier portion is offset from the aperture to enable at least partial emission of the X-ray beam.

The method optionally comprises receiving a product signal from at least one sensor indicative of a detection of the product in the X-ray inspection apparatus; and determining the presence or absence of the product in dependence on the product signal. Determining the presence of a product may be in dependence on the detection of the product by the at least one sensor; and determining the absence of a product may be in dependence on a lack of detection of a product by the at least one sensor for at least a predetermined time.

The method may comprise controlling the actuator mechanism to retain the shutter plate in the open position for a predetermined time window in dependence on the determination of the presence of a product. The method may comprise controlling the actuator mechanism to move the shutter plate to the closed position in dependence on the determination of the absence of a product.

Optionally, the method comprises controlling the X-ray generator to enter a low power state in response to the determination of the absence of a product. Optionally, the method comprises controlling the X-ray generator to resume a full power state from the low power state in response to the determination of the presence of a product.

According to another aspect there is provided computer software which, when executed, is arranged to perform a method according to the aspect above.

### BRIEF DESCRIPTION OF THE DRAWINGS

Embodiments of the invention are further described hereinafter with reference to the accompanying drawings, in which:
Figure 1 is a schematic illustration of an X-ray product inspection system;
Figure 2a shows an X-ray product inspection apparatus 200 according to an embodiment having a shutter plate in an open position;
Figure 2b shows the X-ray product inspection apparatus 200 having a shutter plate in a closed position;
Figure 3a shows an X-ray product inspection apparatus 300 according to another embodiment having a shutter plate in an open position;
Figure 3b shows the X-ray product inspection apparatus 300 having a shutter plate in a closed position;
Figure 4 shows an X-ray product inspection apparatus 400 according to another embodiment in a constructed view;
Figure 5 illustrates the X-ray product inspection apparatus 400 in an exploded view;
Figure 6 shows an X-ray product inspection apparatus according to another embodiment;
Figure 7 shows a schematic illustration of a controller; and
Figure 8 shows a flow chart of a method performed by the controller.

### DETAILED DESCRIPTION

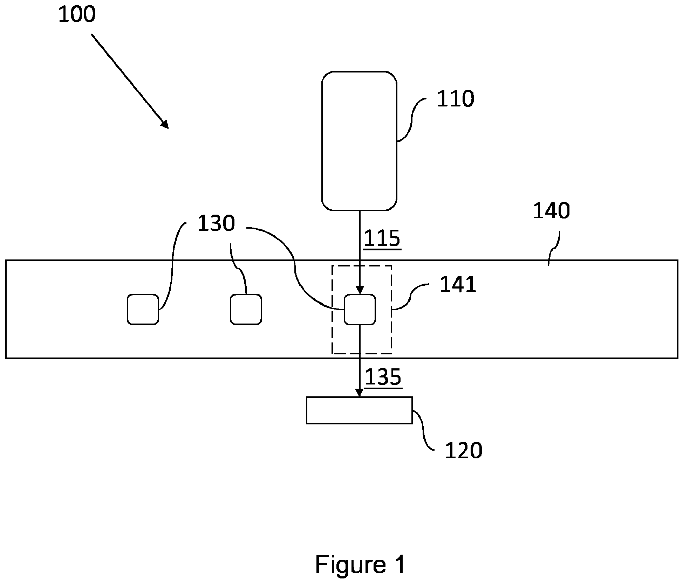

With reference to Figure 1, there is illustrated a schematic of an X-ray product inspection system 100. The system 100 conventionally comprises an X-ray generator 110, such as an X-ray tube, arranged to output an X-ray beam 115. The system 100 further comprises an X-ray detector 120 arranged in a path of the X-ray beam 115. The X-ray detector 120 is configured to detect X-rays in order to generate an image. In use, a product 130 to be inspected is located in the path of the X-ray beam 115 between the generator 110 and the detector 120 such that the X-rays are transmitted through the object 130. The X-ray beam 115 is thus attenuated by the object 130 to form a resultant X-ray beam 135 for detection by the X-ray detector 120. The X-ray detector 120 may then be arranged to generate a resultant image of the internal structure of the object in dependence on the detected X-rays, in order to detect contaminants within the product.

The X-ray product inspection system 100 may be implemented in a production setting, such as a food production setting. The X-ray product inspection system 100 may be implemented as part of an assembly line, and the products 130 may be fed through the system 100 for inspection, such as on a conveyor system 140 arranged to convey products 130 through an inspection zone 141 between the generator 110 and detector 120.

When implementing an X-ray product inspection system such as the system 100, it can be desirable to reduce scatter of the generated X-ray beam, in particular to prevent emission of the X-ray beam outside the system 100.

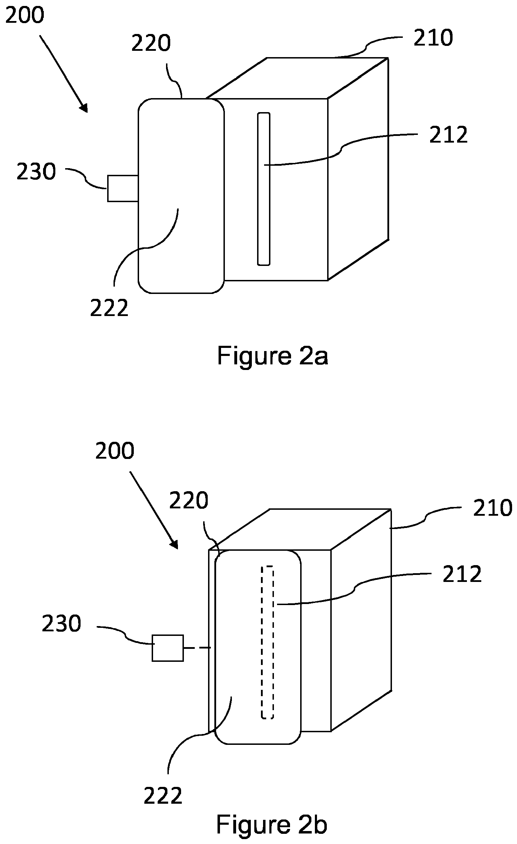

With reference to Figures 2a and 2b, there is shown an X-ray product inspection apparatus 200 according to an embodiment of the invention. The X-ray product inspection apparatus 200 may be implemented as part of a wider product inspection system, such as the product inspection system 100, and may beneficially be implemented to reduce X-ray scatter.

The X-ray product inspection apparatus 200 comprises an X-ray generator 210. The X-ray generator 210 may correspond to the X-ray generator 110 of the system 100. The X-ray generator 210 comprises an aperture 212 for outputting an X-ray beam. That is, the X-ray beam generated by the X-ray generator 210 is emitted, in use, via the aperture 212. The X-ray generator 210 may comprise a collimator for collimating the X-ray beam prior to emission, and thus the aperture 212 may be arranged at an outlet of the collimator.

According to the present invention, scatter of X-rays outside the system 100 or any unwanted emission from the aperture 212 may be reduced in a manner particularly useful to curtainless product inspection systems. A "curtainless" inspection apparatus may be defined as any inspection apparatus without the use of a shielding curtain. Curtainless systems may have particular applicability in the inspection of uncovered food produce or other hygiene sensitive items.

To reduce X-ray scatter, the product inspection apparatus 200 comprises a shutter apparatus arranged, at least partially in use, across the aperture 212. The shutter apparatus comprises a moveable shutter plate 220 having a barrier portion 222 of X-ray attenuating material configured to substantially block the X-ray beam. By "substantially block", it is meant that the barrier portion provides a layer through which most X-rays generated in the X-ray beam are attenuated and thus do not fully penetrate. For example, "substantially block" may be defined as blocking more that 90%, 98% or 99% of X-rays generated, or may be defined as completely blocking the X-ray beam. This may be achieved by providing a threshold thickness of an X-ray attenuating material, wherein the thickness is dependent on the material selected. For example, the barrier portion may comprise a brass layer, a stainless steel layer or a combination of the two. Brass has been shown to perform better than stainless steel at attenuating X-rays, and thus a thinner layer of brass than of stainless steel would be required to achieve the same level of attenuation. However, brass is associated with an increased cost compared to stainless steel. Thus, cost-effective performance can be achieved by providing a layer of stainless steel in combination with a layer of brass, to reduce the thickness of brass required and reduce the cost. For example, according to one embodiment the barrier portion may comprise a 6mm thick layer of brass in addition to a layer of stainless steel, though it will be appreciated that other thicknesses and materials may be used. The thickness of each layer may be tailored depending on the type of X-rays being produced by the X-ray generator 210.

The shutter apparatus comprises an actuator mechanism 230 configured to move the shutter plate between an open position as illustrated in Figure 2a and a closed position as illustrated in Figure 2b. In the open position, the barrier portion 222 is offset from the aperture 212 to enable at least partial emission of the X-ray beam from the aperture 212. In the closed position, the barrier portion 222 is disposed across the aperture 212 to substantially block the X-ray beam.

The moveable shutter plate 220 may be arranged flush with a surface of the X-ray generator 210. In this way, in the closed position the barrier portion 222 is flush with the aperture in order to prevent leakage of X-rays through the sides of the shutter plate 220.

The actuator mechanism 230 may comprise any mechanism suitable for moving the shutter plate 220, in particular any mechanism suitable for converting energy from an energy source, such as mains power, into mechanical motion of the shutter plate 220 in response to a control signal. For example, the actuator mechanism 230 may comprise one or more solenoids which are arranged to move the shutter plate from the closed position to the open position when energised. However, alternative actuator mechanisms can be envisaged, such as a hydraulic, pneumatic or electromechanical actuator, or a linear motor.

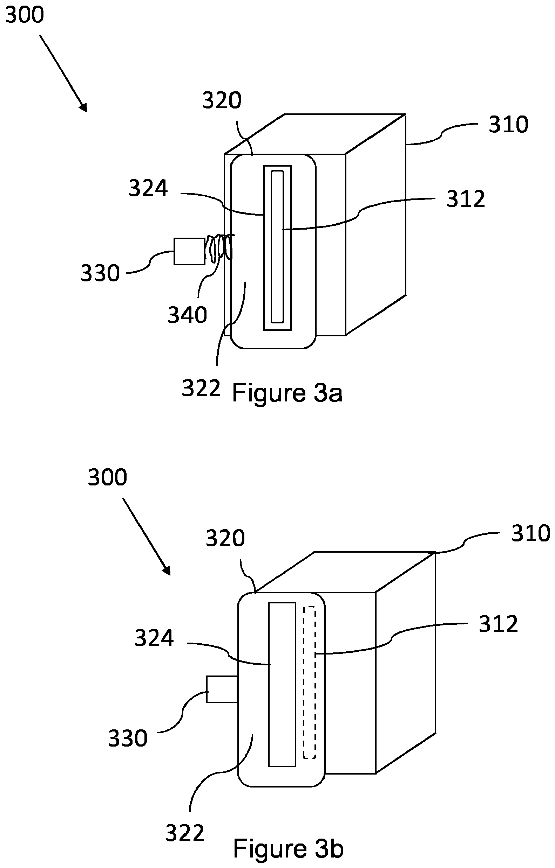

With reference to Figures 3A and 3B, there is shown an X-ray product inspection apparatus 300 according to another embodiment of the invention. Analogously to the apparatus 200, the X-ray product inspection apparatus 300 comprises an X-ray generator 310 having an aperture 312 through which, in use, an X-ray beam is emitted, an actuator mechanism 330 and a movable shutter plate 320. The actuator mechanism 330 is configured to move the shutter plate 320 between an open position as illustrated in Figure 3a and a closed position as illustrated in Figure 3b.

The movable shutter plate 320 comprises a barrier portion 322 which may be composed analogously to the barrier portion 222. In the closed position shown in Figure 3b, the barrier portion 322 is disposed across the aperture 312 to substantially block the X-ray beam as described with reference to Figure 2b.

The movable shutter plate 320 differs from the shutter plate 220 in that the movable shutter plate 320 comprises an aperture portion 324 at least partially permeable to the X-ray beam. For example, the aperture portion 324 may be a hole, inlet or the like through the shutter plate 320. In other embodiments, the aperture portion 324 may comprise one or more layers of material which are permeable or partially permeable to the X-ray beam. That is, the aperture portion 324 is arranged to attenuate the X-ray beam less than the barrier portion 322. In the open position shown in Figure 3a, the aperture portion 324 aligns at least partially with the aperture 312 to enable at least partial emission of the X-ray beam through the aperture portion 324. In particular embodiments, the aperture portion 324 may align fully with the aperture 312 such that the full extent of the aperture 312 is aligned with the aperture portion 324. In contrast, in the embodiment shown in Figures 2a and 2b, in the open position the movable shutter plate 220 must be completely displaced from the aperture 212. Beneficially by implementing the aperture portion 324, the open position can be more tightly controlled.

The aperture portion 324 may have a corresponding shape to the aperture 312 of the generator 310 in order to enable efficient passage of the X-ray beam through the aperture portion 324 whilst also minimising the necessary size of the shutter plate 320. The barrier portion 322 may be arranged to surround the aperture portion 324. Beneficially, this means that displacing the shutter plate 320 in any direction from the open position causes the shutter apparatus to be in a closed position. This may be beneficial, for example if the actuator 330 malfunctions, as it increases the likelihood of the movable shutter plate 320 resting in a closed position, preventing X-ray emission.

The shutter apparatus may also comprise a biasing mechanism 340 as illustrated in Figure 3a. It will be appreciated that the biasing mechanism 340 may also be used in conjunction with the product inspection apparatus 200 shown in Figures 2a and 2b.

The biasing mechanism 340 is configured to bias the movable shutter plate 320 to the closed position shown in Figure 3b. That is, in the absence of the actuator mechanism 330 being energised or otherwise activated, the movable shutter plate 320 is arranged to return to the closed position under the action of the biasing mechanism 340. For example, the biasing mechanism 340 may comprise one or more springs 340 connecting the movable shutter plate 322 to a fixed point on the product inspection apparatus 300, however it will be appreciated that any biasing mechanism may be implemented. Thus, beneficially in the absence of activating the actuator 330, by default the movable shutter plate 320 remains closed and the barrier portion 322 effectively blocks the X-ray beam from emission.

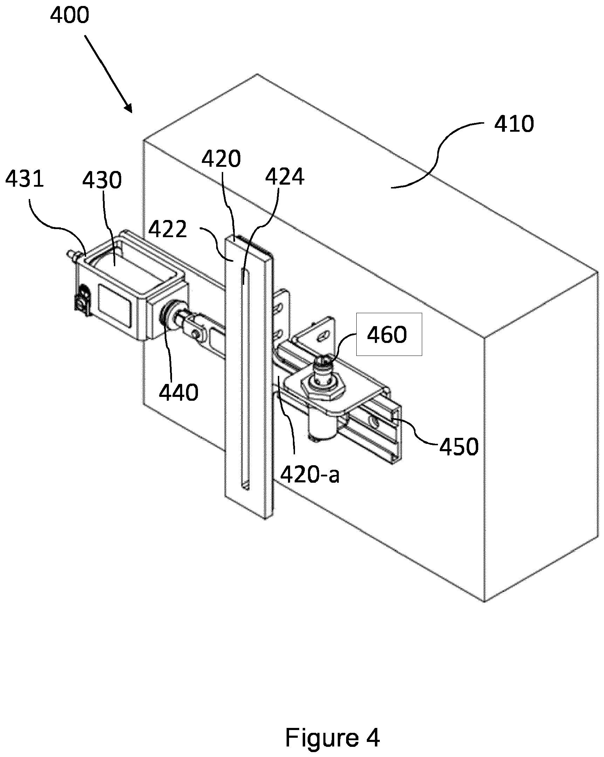

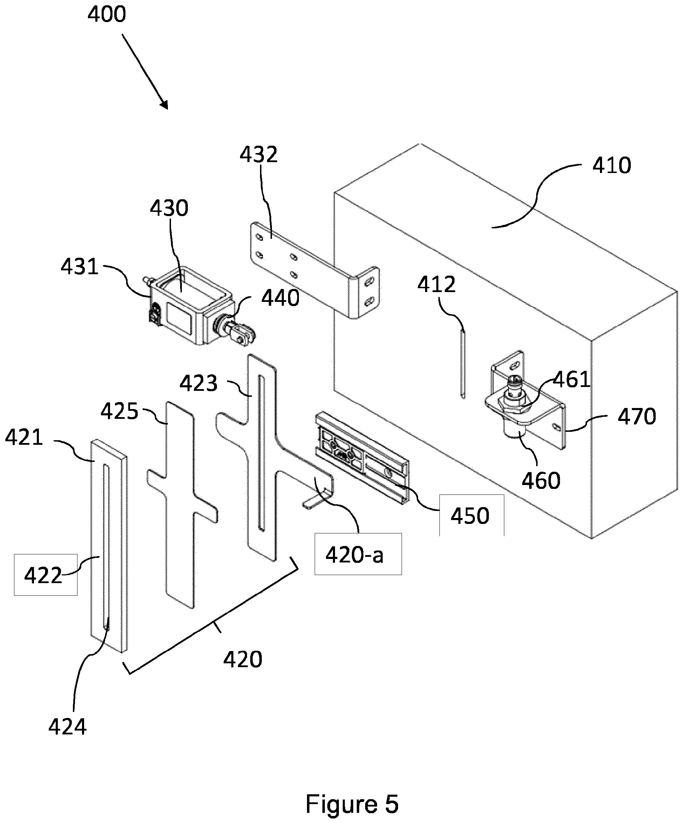

With reference to Figures 4 and 5, there is illustrated an X-ray product inspection apparatus 400 according to an embodiment of the invention. Figure 4 shows the product inspection apparatus 400 in a constructed view, and Figure 5 shows the product inspection apparatus in an exploded view.

The X-ray product inspection apparatus 400 comprises an X-ray generator 410 having an aperture 412 for outputting an X-ray beam, and a shutter apparatus. The shutter apparatus comprises a moveable shutter plate 420 comprising a barrier portion 422 and an aperture portion 424, as described with reference to the embodiment shown in Figures 3a and 3b. The shutter apparatus comprises an actuator mechanism 430 in the form of a solenoid 430 contained within a housing 431. When the solenoid 430 is energised, the shutter plate 420 is pushed from a closed position to an open position as described previously. The solenoid 430 is disposed in a housing 431 which is in use, affixed to a mount 432 to secure the solenoid 430 in a fixed position on the apparatus 400. The movable shutter plate 420 is secured to a first end of a biasing mechanism 440 in the form of a spring 440. A second end of the spring 440 is secured to the housing 431. In this way, when the solenoid 430 is energised, the resultant magnetic force acting on the movable shutter plate 420 overcomes the biasing force provided by the spring 440 and causes the shutter plate 420 to be pushed to the open position. When the solenoid is de-energised, the magnetic force is removed, and the biasing force provided by the spring 440 causes the shutter plate to be pulled back to the closed position. Thus, when power is removed, the apparatus 400 fails safe into the closed position.

To facilitate and control the movement of the shutter plate 420 between the open and the closed positions, the shutter apparatus may be provided with a linear bearing 450 secured to a fixed point of the product inspection apparatus 400. The movable shutter plate 420 can then be provided with an arm portion 420-a shaped to fit into the linear bearing 450 and move along the linear bearing 450 as the plate 420 is moved between the open position and the closed position. That is, the linear bearing 450 defines a path for the arm portion 420-a and thus limits the movement of the shutter plate 420 to a fixed trajectory.

The shutter apparatus 400 may further be provided with a proximity sensor 460 arranged to detect whether the shutter plate 420 is in the open or closed position. In the illustrated embodiment, the proximity sensor 460 is mounted to a bracket 470 at a fixed location of the inspection apparatus 400 with securing means 461. The proximity sensor 460 is in the illustrated embodiment located along the linear bearing 450 and arranged to detect the arm portion 420-a of the shutter plate 420 when in the open position. However, it will be appreciated that the proximity sensor 460 may be located elsewhere and may be arranged to detect different portions of the movable shutter plate 420.

With reference to Figure 5, it can be seen that the shutter plate 420 may comprise a plurality of layers, which are illustrated separately in the exploded view. As discussed, the shutter plate 420 may comprise a brass layer 421 and a stainless steel layer 423 in the barrier portion 422 arranged to substantially block the X-ray beam. Each of the brass layer 421 and stainless steel layer 423 comprise a gap at the aperture portion 424 to enable at least partial emission of the X-ray beam. The shutter plate 420 further comprises in some embodiments a filter layer 425 of material extending across the aperture portion 424. The filter layer 425 may be configured to attenuate a portion of the X-ray beam below an energy threshold, i.e., attenuate soft X-rays. Low energy or "soft" X-rays are more susceptible to scatter widely and thus it is beneficial to attenuate this portion of the beam even when the shutter plate is in the open position. The filter layer 425 may be formed from any material suitable for attenuating soft X-rays, such as aluminium.

According to some embodiments, the shutter apparatus may be controlled in an automated manner such that the movable shutter plate is only retained in the open position when it is likely a product is being inspected. In this way, the risk of unwanted scatter from the X-ray generator can be reduced.

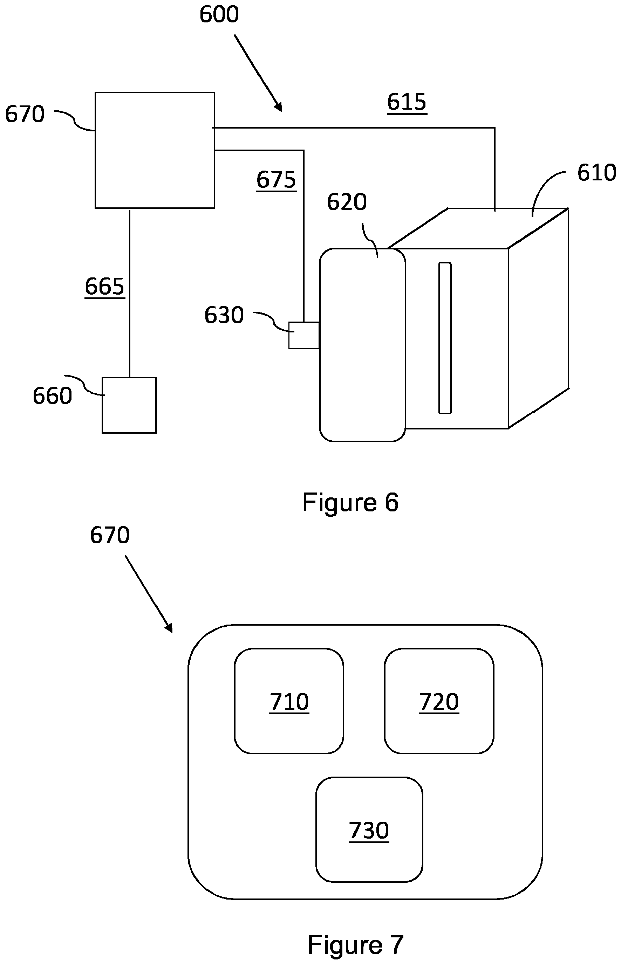

With reference to Figure 6, there is shown an X-ray product inspection apparatus 600 for facilitating automated control of a shutter plate. The X-ray product inspection apparatus 600 comprises an X-ray generator 610 and a shutter apparatus comprising a movable shutter plate 620 and an actuator 630. The generator 610 and shutter apparatus may be arranged as described with reference to any of the preceding embodiments shown in Figures 2a to 5.

The X-ray product inspection apparatus 600 comprises at least one sensor 660 configured to detect a product in a product inspection system. For example, when the apparatus 600 is implemented in the product inspection system 100, the sensor 660 may be disposed along the conveyor system 140 on the approach to the apparatus 600. In this way, the sensor 660 is arranged to detect the presence of a product 130 about to be inspected by the apparatus 600. The sensor 660 may comprise any sensing technology capable of detecting a product, such as a camera, a proximity sensor, or the like. Whilst a single sensor 660 is illustrated, it will be appreciated that the X-ray product inspection apparatus 600 may comprise a plurality of sensors 660 of the same or different types, such as arranged in a sensor array.

The X-ray product inspection apparatus 600 comprises a controller 670. A schematic illustration of the controller 670 is shown in Figure 7.

The controller 670 comprises at least one processor 710, at least one memory device 720 and a communication module 730 arranged to communicate with other components of the apparatus 600, as will be explained. The at least one memory device 720 is arranged to store computer-readable instructions which when executed by the at least on processor 710, cause the at least one processor 710 to determine a presence or absence of a product to be inspected in the apparatus 600, and determine a control signal for controlling the actuator 630 to move the shutter plate between the closed position and the open position in dependence on the determination.

The communication module 730 of the controller is communicably coupled to each of the sensor 660 and to the actuator 630. Each of the sensor 660 and the actuator 630 may be arranged to communicate with the communication module 730 through a wired connection via one or more electrical inputs and outputs. In some embodiments, each of the sensor 660 and actuator 630 may be arranged to communicate with the communication module via wireless communication. The wireless communication may be a short-range wireless communication protocol such as Bluetooth, NFC, WiFi or the like, or may comprise an indirect wireless communication through a network such as a local area network (LAN) or the Internet.

The sensor 660 is configured to transmit a product signal 665 indicative of a detected product or lack thereof to the controller 670. The controller 670 is arranged to determine whether a product is present or absent in dependence on the product signal 665 received from the sensor 660. For example, the controller 670 may be arranged to determine that a product is present in dependence on the product signal 665 indicating that a product has been detected by the sensor 660. The controller 670 may furthermore determine the absence of a product in dependence on the product signal 665 indicating a lack of detection of a product by the sensor 660 for at least a predetermined time, such as 10 seconds, 15 seconds or 20 seconds. The predetermined time may be defined in dependence on the location of the sensor 660, in particular in dependence on the time taken for a product passing through the system to progress from the sensor 660 to the path of the X-ray beam generated by the X-ray generator 610. Thus, the predetermined time may be tailored to allow sufficient time between detection by the sensor 660 and the completion of inspection.

The controller 670 is configured to output a control signal 675 to the actuator 630 to move the shutter plate 620 between the closed position and the open position in dependence on the determination. For example, if it is determined that a product is present, the control signal 675 may be output to control the actuator mechanism 630 to retain the shutter plate 620 in the open position for a predetermined time window. The predetermined time window may be defined as sufficient for the product to pass the X-ray beam output by the generator 610, such as 5 seconds, 10 seconds, or 15 seconds for example. It will be appreciated that the time window may depend on the location of the sensor 660 and the speed at which a product passes through the apparatus 600. If it is determined that a product is absent, the control signal 675 may be output to control the actuator mechanism 630 to move the shutter plate to the closed position, e.g., by deenergising the solenoid. It will be appreciated that in some embodiments, the absence of a product may result in the absence of a control signal 675, and the actuator mechanism 630 may be arranged to power down or de-energise the solenoid if a control signal 675 has not been received after the predetermined time window, thus causing the shutter plate to move to the closed position.

According to some embodiments, the control of the moveable shutter plate may be implemented in conjunction with control of a power state of the X-ray generator 610. The controller 670 may be further communicably coupled to the X-ray generator 610 and arranged to transmit a control signal 615 to the X-ray generator for controlling the X-ray generator 610 to transition between a low power state and a full power state. The low power state may be defined as an idle mode sufficient for the X-ray generator 610 to remain running at a minimum power level. For example, the low power state may be defined as a power level of less than 50W, less than 40W or less than 45W. Thus, when the X-ray beam is not required, the power consumption of the system can be reduced as well as further reducing undesired X-ray scatter. In contrast to fully powering off the X-ray generator 610, keeping the generator running at a low power facilitates the ability to return to a full power state rapidly. Furthermore, repeatedly powering up and down completely causes degradation of the X-ray generator. Maintaining the generator in a low power state helps to reduce this degradation. Furthermore, reducing the power state of the generator to a low state helps to extend the life of the detector by reducing unnecessary illumination by X-ray beams on the detector.

The controller 670 may thus be configured to output the control signal 615 to the X-ray generator 610 to enter a low power state when the absence of a product is determined. Then, when the controller 670 determines that a product is present, the control signal 615 may be output to control the X-ray generator 610 to resume a full power state. In this way, the power state of the X-ray generator 610 may be co-ordinated with the location of the shutter plate 620, such that when the shutter plate 620 is in the closed position, the X-ray generator 610 is in a low power state to further reduce the risk of X-ray emission.

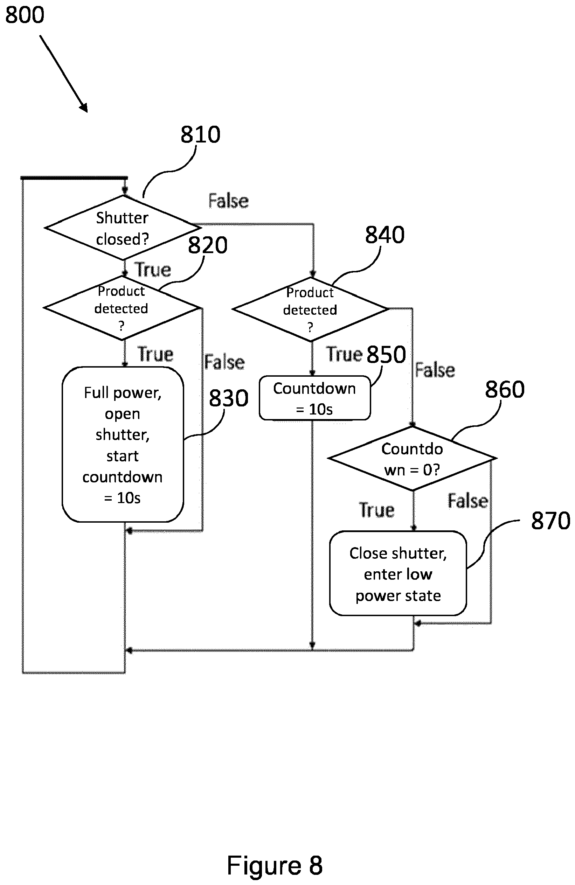

With reference to Figure 8, there is shown a flow chart of a method 800 which may be implemented by the controller 670 according to one embodiment.

In step 810, it is determined whether the shutter plate 620 is in the closed position. This may be determined in dependence on a signal received from a proximity sensor such as the proximity sensor 460 or may be otherwise inferred from the status of the actuator mechanism 630.

If the shutter plate 620 is in the closed position, in step 820 it is determined whether a product is detected by the sensor 660. If a product is detected, the method progresses to step 830. In step 830, a control signal 675 is output to move the shutter plate 620 to the open position and optionally, the control signal 615 may be output to the X-ray generator to resume a full power state. A countdown timer is started for the predetermined time window, in this example 10 seconds, and the method returns to step 810. If no product is detected in step 820, the method proceeds directly back to step 810.

If the shutter plate is determined to be in the open position in step 810, the method proceeds to step 840. In step 840 it is determined whether a product is detected by the sensor 660. If a product is detected, the method progresses to step 850. In step 850, a countdown timer is started (or re-started) for the predetermined time window, in this example 10 seconds, and the method returns to step 810.

If it is determined that no product is detected in step 840, the method proceeds to step 860. In step 860 it is determined whether all timers running have expired. If the timer(s) have not yet expired, the method returns to step 810. If the timer(s) have all expired, the method proceeds to step 870. In step 870, a control signal 675 is output to move the shutter plate 620 to the closed position and optionally, the control signal 615 may be output to the X-ray generator 610 to enter a low power state.

Thus, the present invention provides a mechanism by which undesired X-ray scatter from a product inspection apparatus can be reduced with the implementation of a shutter apparatus to reduce the risk of an X-ray beam being emitted in circumstances where no product is currently being inspected. As described, this aspect may be combined with the implementation of a low power state for the X-ray generator to further reduce X-ray scatter in addition to reducing power consumption.

It will be appreciated that embodiments of the present invention can be realised in the form of hardware, software or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape. It will be appreciated that the storage devices and storage media are embodiments of machine-readable storage that are suitable for storing a program or programs that, when executed, implement embodiments of the present invention. Accordingly, embodiments provide a program comprising code for implementing a system or method as claimed in any preceding claim and a machine readable storage storing such a program. Still further, embodiments of the present invention may be conveyed electronically via any medium such as a communication signal carried over a wired or wireless connection and embodiments suitably encompass the same.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

## Claims

1. An X-ray product inspection apparatus (200), comprising:
an X-ray generator (210) comprising an aperture (212) for outputting an X-ray beam;
a shutter apparatus arranged across the aperture, the shutter apparatus comprising:
a moveable shutter plate (220) having a barrier portion (212) of X-ray attenuating material configured to substantially block the X-ray beam; and
an actuator mechanism (230) configured to move the shutter plate (220) between a closed position in which the barrier portion is disposed across the aperture (212) to substantially block the X-ray beam and an open position in which the barrier portion is offset from the aperture (212) to enable at least partial emission of the X-ray beam.

2. The X-ray product inspection apparatus (200) of claim 1, further comprising a controller configured to:
determine a presence or absence of a product to be inspected in the X-ray inspection apparatus; and
output a control signal to the actuator mechanism (230) to move the shutter plate (220) between the closed position and the open position in dependence on the determination; the apparatus further comprising at least one sensor configured to detect the product in the X-ray inspection apparatus and provide a product signal indicative of the detection to the controller,
wherein the controller is configured to determine the presence or absence of the product in dependence on the product signal.

3. The X-ray product inspection apparatus (200) of claim 2, wherein the controller is configured to:
determine the presence of a product in dependence on the detection of the product by the at least one sensor; and
determine the absence of a product in dependence on a lack of detection of a product by the at least one sensor for at least a predetermined time.

4. The X-ray product inspection apparatus (200) of any of claims 2 to 3, wherein the controller is configured to output a control signal to the actuator mechanism (230) to retain the shutter plate (220) in the open position for a predetermined time window in dependence on the determination of the presence of a product.

5. The X-ray product inspection apparatus (200) of any of claims 2 to 4, wherein the controller is configured to output a control signal to the actuator mechanism (230) to move the shutter plate to the closed position in dependence on the determination of the absence of a product.

6. The X-ray product inspection apparatus (200) of any of claims 2 to 5, wherein the controller is configured to output a control signal to the X-ray generator (210) to enter a low power state in response to the determination of the absence of a product, optionally wherein the controller is configured to output a control signal to the X-ray generator (210) to resume a full power state from the low power state in response to the determination of the presence of a product.

7. The X-ray product inspection apparatus (300) of any preceding claim, wherein the movable shutter plate (320) comprises an aperture portion (324) at least partially permeable to the X-ray beam, and wherein in the open position the aperture portion (324) aligns with the aperture (312) to enable at least partial emission of the X-ray beam through the aperture portion (324), optionally wherein the shutter plate (320) comprises a filter layer (425) of material across the aperture portion configured to attenuate a portion of the X-ray beam below an energy threshold.

8. The X-ray product inspection apparatus (200) of any preceding claim, wherein the barrier portion (222) comprises a layer of brass.

9. The X-ray product inspection apparatus (300) of any preceding claim, wherein the shutter apparatus comprises a biasing mechanism (340) configured to bias the shutter plate to the closed position, optionally wherein the biasing mechanism (340) comprises a spring connected to the shutter plate.

10. The X-ray product inspection apparatus (200) of any preceding claim, wherein the actuator mechanism (230) comprises one or more solenoids which are arranged to move the shutter plate (220) from the closed position to the open position when energised.

11. The X-ray product inspection apparatus (400) of any preceding claim, wherein the shutter apparatus comprises a linear bearing (450), and wherein the shutter plate comprises an arm portion (420-a) arranged to move along the linear bearing between the open position and the closed position.

12. The X-ray product inspection apparatus (400) of any preceding claim, wherein the shutter apparatus comprises a proximity sensor (460) arranged to detect whether the shutter plate is in the open position or the closed position.

13. The X-ray product inspection apparatus (200) of any preceding claim, wherein the X-ray generator (210) comprises a collimator for collimating the X-ray beam.

14. A computer-implemented method for controlling an X-ray product inspection apparatus (200) comprising an X-ray generator (210) having an aperture (212) for outputting an X-ray beam, the method comprising:
determining a presence or absence of a product to be inspected in the X-ray inspection apparatus (200); and
in dependence on the determination, controlling an actuator mechanism (230) to move a shutter plate (220) across the aperture (212) between a closed position in which a barrier portion (222) of the shutter plate is disposed across the aperture to substantially block the X-ray beam and an open position in which the barrier portion (222) is offset from the aperture to enable at least partial emission of the X-ray beam.

15. Computer software which, when executed, is arranged to perform a method according to claim 14.
